# EUROPÄISCHE PATENTANMELDUNG

(11) **EP 3 437 684 A1**
(43) Veröffentlichungstag der Anmeldung: **06.02.2019**
(21) Anmeldenummer: 18000634.8
(22) Anmeldetag: 30.07.2018
(51) Int. Cl.: A61M 16/00, A61M 16/06

(54) **VERFAHREN UND VORRICHTUNG ZUR BEHANDLUNG VON ATEMSTÖRUNGEN**

(30) Priorität: 01.08.2017 CH 10042017
(71) Anmelder: Löwenstein Medical Technology S.A., 2557 Luxembourg (LU); Nirkko, Arto Christian, 6045 Meggen (CH)
(72) Erfinder: Nirkko, Arto Christian, CH-6045 Meggen (CH)
(74) Vertreter: Marx, Thomas

(57) **Zusammenfassung**

Es gibt einerseits obstruktiv bedingte nächtliche Atemstörungen (obstruktive Schlafapnoe), andererseits zentrale Atemstörungen (Cheyne Stokes Atmung und zentrale Schlafapnoe), welche infolge periodischen Oszillationen des zentralen Atemantriebs im Atemzentrum des Himstamms Zustandekommen. Die obstruktive Schlafapnoe wird meist durch kontinuierlichen positiven Luftwegs-Druck (CPAP, appliziert mittels Gerät, Schlauch und Maske) behandelt. Die bisherige Behandlung der zentralen Atemstörungen mittels einer Form der Beatmung, der Antizyklischen / Adaptiven Servo-Ventilation (ASV), wurde durch Studienresultate in Frage gestellt, welche eine hierdurch erhöhte Sterblichkeit bei häufigen Patientengruppen nahelegen. Die Erfindung versucht, das Problem anstatt mittels forcierter passiver Beatmung während den Phasen mit reduziertem Atemantrieb, stattdessen dadurch anzugehen, dass auch in den Phasen mit reduziertem oder fehlendem Atemantrieb eigene aktive Atemzüge ausgelöst werden. Dies wird durch kurze Druckimpulse oder Druckstufen bewirkt, welche nicht zur Beatmung ausreichen, aber jewiels über einen Mechanorezeptor-vermittelten Stimulus einen Atemzug triggern.

## Beschreibung

Die vorliegende Erfindung bezieht sich auf eine Vorrichtung mit erweiterten Verfahrensmerkmalen, welche zur Behandlung oder Ergänzung der Behandlung von Patienten mit Atemstörungen benutzt werden kann, was mittels Auslösen / Anstossen von eigenen Atemzügen durch Anwendung von Druckimpulsen, Druckrampen oder Druckstufen, kurz gesagt kurzzeitigen Druckänderungen bewirkt wird, welche die Mechanorezeptoren von Thorax, Lunge oder Atemwegen stimulieren sollen, gemäss Patentanspüchen 1 bis 8. Aufgrund der Vorrichtungsmerkmale soll eine Behandlung insbesondere von gemischt obstruktiv-zentralen und rein zentralen nächtlichen Atemstörungen im Sinne von Cheyne-Stokes-Atmung bis hin zum zentralen Schlafapnoe-Syndrom erleichtert oder ermöglicht werden. Dies ist wichtig insbesondere in Anbetracht von neueren Studienresultaten, welche darauf hinweisen, dass die bisherige Standard-Therapie dieser Atemstörungen bei manchen Patientengruppen zu einer erhöhten Sterblichkeit führen könnte und deshalb bei diesen Patienten vermieden werden sollte.

### Stand der Technik:

Nächtliche Atemstörungen mit Atemausetzem (Apnoen) und Atemabschwächungen (Hypopnoen) kommen einerseits infolge periodischer Einengung / Verschluss (Obstruktion) vornehmlich der oberen Atemwege vor (obstruktives Schlafapnoe-Syndrom), und andererseits auch infolge periodischem An- und Abschwellen des zentralen Atemantriebs im Hirnstamm (Schwankungen Im Rahmen der Cheyne-Stokes-Atmung) bis hin zu dessen periodischen vollständigen Pausieren (zentrales Schlafapnoe-Syndrom). Es kommen auch gemischte Formen vor (gemischtes obstruktiv-zentrales Schlafapnoe-Syndrom). Ein relevantes obstruktives Schlafapnoe-Syndrom (oSAS) wird meist mittels kontinuierlichem positiven Atemwegs-Druck (continuous positive airway pressure, CPAP) während dem Nachtschlaf behandelt, welcher über eine Maske (Nasenmaske bis hin zur Ganzgesichtsmaske, in speziellen Situationen auch Tubus in der Luftröhre) und einen Schlauch von einem Gerät appliziert wird. Eine Cheyne-Stokes-Atmung, eine zentrale Schlafapnoe, oder die zentrale Komponente eines gemischten Schlafapnoe-Syndroms kann mit einem mehr oder weniger konstanten Druck aber ungenügend oder gar nicht behandelt werden, und kann sich im Gegenteil dadurch sogar verschlechtern. Zur Behandlung dieser Schlafapnoe-Formen wurde bisher meist die antizyklische oder adaptive Servo-Ventilation (ASV) angewandt, welche in den Phasen von fehlendem Atemantrieb effektiv eine externe Beatmung durchführt, d.h. bei Jedem Atemzug den Druck ändert, Indem der zur Einatmung notwendige vollen Druck appliziert wird, und der Druck beim Ausatmen entsprechend reduziert wird. Diese Druckunterschiede werden in Phasen mit zentral (vom Atemzentrum her) zuviel und zuwenig angesteuerter Atmung jeweils umgekehrt ("antizyklisch") angepasst, um die Atmung insgesamt über längere Zeitabschnitte konstant zu halten.

Dies funktioniert technisch recht gut, aber kürzliche Studienresultate weisen darauf hin, dass diese ASV bei Patienten mit Herzinsuffizienz wahrscheinlich zu einer erhöhten Sterblichkeit führt. Man versucht daher, auf andere nächtliche Beatmungs-Modalitäten umzusteigen, aber diese funktioneren schlechter oder verstärken die zentrale Komponente eben eher noch. Zudem haben gerade eben Patienten mit zentralem Schlafapnoe-Syndrom häufig eine Herzinsuffizienz, weil diese eine Cheyne-Stokes-Atmung und eine zentrale Schlafapnoe begünstigt. Gefragt ist also eine Alternative, welche ohne die hohen und anscheinend schädlichen Beatmungsdruckwerte auskommt.

### Beschreibung der Erfindung und deren Merkmale:

Die vorliegende Erfindung umgeht das Problem der hohen Beatmungsdruckwerte, indem die Vorrichtung nicht wie bisher üblich versuchen soll, den Patienten forciert passiv zu beatmen, währenddem sein Atemzentrum gar keinen Bedarf zur Atmung hat, sondern stattdessen versuchen soll, die eigene Atmung über einen Reflexmechanismus derart anzuregen, dass der Patient als Antwort auf einen Stimulus ohne relevante weitere Druckunterstützung jeweils selbst aktiv einen Atemzug ausführt. Dadurch verschlechtern sich in dieser Phase mit vermindertem Atemantrieb auch die Blutgase weniger stark, und die nächste spontane Phase mit vermehrtem Atemantrieb überschiesst dann nicht derart stark, dass die nächste Pause durch Rückkoppelung wieder umso stärker gefördert wird, wie das bei der Cheyne-Stokes-Atmung sonst der Fall ist (d.h. das oszillatorische Aufschaukeln wird geddämpft, wenn während der Atempause doch selbst etwas mehr geatmet wird).

Nebst Chemorezeptoren im Himstamm, welche die Blutgas-Konzentration messen (v.a. Kohlendioxid/CO2 und Sauerstoff/O2), beeinflussen auch Mechanorezeptoren in Thorax, Lunge und Atemwegen die Steuerung der Atmung. Diese Mechanorezeptoren sollen im Rahmen der Erfindung durch kurzdauernde und niederamplitudige Druckimpulse angeregt werden, und so jeweils einen Atemzug triggern. Wie auch bei bisherigen Geräten alle möglichen Geräteparameter separat einstellbar sind, so sollen auch die Druckimpulse für Jeden Patienten einstellbar sein, und können in kurzen Druckrampen, Druckstufen oder Druckimpulsen sowohl in positive Richtung (mehr Druck) wie oder auch in negative Richtung (weniger Druck) appliziert werden, je nach Situation und Kombination mit obstruktiven Schlafapnoe-Komponenten und anderen Faktoren.

Als Ausführungsbeispiel wird ein kommerziell erhältiches CPAP- oder BiPAP-Gerät (bilevel-PAP) angenommen, welchem die zusätzliche Fähigkeit einprogrammiert wird, bei jedem spontanen beginnenden Atemzug den Druck mit einem kurzen Puls während z.B. 1 sec um z.B. 3 mbar zu senken und dann auf den ursprünglichen Druck zurückzukehren, und dies auch in Abwesenheit von spontanen Atemzügen wahrend einer vollständigen Atempause mit einer Frequenz von minimal 15/min weiterhin zu tun. Ähnliche Fähigkeiten zum passiven Unterstützen von ungenügenden eigenen Atemzügen, oder zum vollständigen Ersatz von eigenen Atemzügen während einer Apnoe gibt es bei heutigen Geräten schon. Im Unterschied dazu soll es bei der Erfindung nicht zu einer relevanten Unterstützung oder Ersatz des Atemzugs kommen, sondern (zusätzlich dazu oder alleinig) zum Auslösen oder Verstärken eines eigenen aktiven Atemzuges durch einen natürlich vorhandenen Reflexmechanismus, mit kurzen und schärferen Druckänderungen, welche aber weniger Druck applizieren und weniger lange dauern. Dies ist mit heute erhältlichen Geräten nicht gezielt möglich, allenfalls andeutungsweise als Nebeneffekt durch Einstellung mit Parametem am Rande eines anderen heute beabsichtigten Beatmungsmodus.

## Patentansprüche

1. Verfahren zur Auslösung oder Verstärkung von eigenen Atemzügen bei Patienten mit Atemstörungen, **dadurch gekennzeichnet, dass** kurzzeitige Änderungen im Atemdruck in den Luftwegen appliziert werden, welche genannten Effekt reflektorisch bewerkstelligen sollen.

2. Verfahren gemäss Patentanspruch 1, **dadurch gekennzeichnet, dass** es sich um eine kurzzeitige Zunahme des Drucks handelt, welcher appliziert wird.

3. Verfahren gemäss Patentanspruch 1, **dadurch gekennzeichnet, dass** es sich um eine kurzzeitige Abnahme des Drucks handelt, welcher appliziert wird.

4. Verfahren gemäss Patentanspruch 1 bis 3, **dadurch gekennzeichnet, dass** es sich um eine in kurzer Zeit vorgenommene Änderung (Abnahme oder Zunahme) des Drucks handelt, welche länger aufrechterhalten wird und später zurückgesetzt wird oder in der Zeit bis zum nächsten Atemzug zurück auf den Ausgangswert abklingt (zum Beispiel sogenanntes Sägezahn-Zeitprofil).

5. Vorrichtung zur Ausführung eines der Verfahren gemäss Patentanspruch 1 bis 4, **dadurch gekennzeichnet, dass** die Druckänderungen in einem Gerät erzeugt werden und über Schlauch und/oder Maske den Atemwegen zugeführt werden.

6. Vorrichtung zur Ausführung eines der Verfahren gemäss Patentanspruch 1 bis 5, **dadurch gekennzeichnet, dass** ein Gerät, welches auch andere Fähigkeiten zur Behandlung mittels Druckapplikation zu den Atemwegen besitzt, mit den beschriebenen Fähigkeiten zusätzlich ausgestattet wird.

7. Anwendung von Verfahren gemäss Patentanspüchen 1 bis 4 an Patienten mit nächtlichen Atemstörungen.

8. Anwendung einer Vorrichtung gemäss Patentanspruch 5 oder 6 an Patienten mit nächtlichen Atemstörungen.
